# EUROPÄISCHE PATENTSCHRIFT

(11) **EP 2 920 854 B1**
(45) Veröffentlichungstag und Bekanntmachung des Hinweises auf die Patenterteilung: **23.11.2016**
(21) Anmeldenummer: 13791933.8
(22) Anmeldetag: 07.11.2013
(51) Int. Cl.: H01R 39/64, F16M 13/02, H01B 11/18

(54) **KOAXIALKABEL ZUR ELEKTRISCHEN ÜBERTRAGUNG EINES HOCHFREQUENZ- UND/ODER HOCHGESCHWINDIGKEITS-DATENSIGNALS, DREHKUPPLUNG MIT ZWEI DERARTIGEN KOAXIALKABELN, SOWIE HALTEVORRICHTUNG MIT WENIGSTENS EINER SOLCHEN DREHKUPPLUNG**
COAXIAL CABLE FOR THE ELECTRICAL TRANSMISSION OF A RADIOFREQUENCY AND/OR HIGH-SPEED DATA SIGNAL, ROTATING JOINT COMPRISING TWO SUCH COAXIAL CABLES, AND RETAINING APPARATUS COMPRISING AT LEAST ONE SUCH ROTATING JOINT
CÂBLE COAXIAL POUR LA TRANSMISSION ÉLECTRIQUE D'UN SIGNAL DE DONNÉES À HAUTE FRÉQUENCE ET/OU À GRANDE VITESSE, ACCOUPLEMENT ROTATIF DE DEUX CÂBLES COAXIAUX DE CE TYPE, AINSI QUE DISPOSITIF DE RETENUE COMPRENANT AU MOINS UN TEL ACCOUPLEMENT ROTATIF

(30) Priorität: 13.11.2012 DE 202012010854 U
(43) Veröffentlichungstag der Anmeldung: 23.09.2015
(73) Patentinhaber: Ondal Medical Systems GmbH, 36088 Hünfeld (DE)
(72) Erfinder: GÖBEL, Andreas, 36132Eiterfeld (DE); ICKLER, Fritz, 36275 Kirchheim (DE)
(74) Vertreter: Graf von Stosch, Andreas
(86) Internationale Anmeldenummer: PCT/EP2013/003354
(87) Internationale Veröffentlichungsnummer: WO 2014/075780

(56) Entgegenhaltungen:
- EP-A1- 2 309 608
- EP-A2- 0 170 159
- WO-A1-2011/151081
- US-A1- 2004 251 390

## Beschreibung

Die vorliegende Erfindung betrifft ein Koaxialkabel zur elektrischen Übertragung eines Hochfrequenz- und/oder Hochgeschwindigkeits-Datensignals, insbesondere für industrielle und medizinisch-technische Anwendungen. Ferner betrifft die Erfindung auch eine Drehkupplung mit zwei derartigen Koaxialkabeln, sowie eine Haltevorrichtung mit wenigstens einer solchen Drehkupplung.

Im medizinisch-technischen Bereich ist es oft erforderlich, Hochfrequenz- und/oder Hochgeschwindigkeits-Datensignale von einem Gerät zu einem anderen zu übertragen. Bei solchen Geräten handelt es sich beispielsweise um eine Rechnereinheit, etwa einen Computer, um einen Sensor, zum Beispiel zum Erfassen von Herzkreislaufparameter eines Patienten, um eine Kamera jeglicher Art, zum Beispiel zur Lifeübertragung von Operationen, um Geräte der bildgebenden Verfahren, zum Beispiel Röntgengeräte, CT Scanner, MRT Geräte oder Ultraschallgeräte, oder um eine Anzeigeeinheit, beispielsweise einen Monitor.

Im Zusammenhang mit der vorliegenden Erfindung werden unter der Bezeichnung "Hochfrequenz-Datensignate" allgemein Radiofrequenzsignale verstanden, insbesondere jedoch Radiofrequenzsignale mit Frequenzen im UHF-Bereich oder darüber, d.h. elektromagnetische Signale mit einer Frequenz von etwa 300 MHz und höher (die UHF-Bandbreite reicht bis etwa 3 GHz). Bevorzugt umfassen "Hochfrequenz-Datensignale" auch Radiofrequenzsignale mit Frequenzen im SHF-Bereich, d.h. bis zu etwa 30 GHz, und besonders bevorzugt sogar Radiofrequenzsignale mit Frequenzen im EHF-Bereich, d.h. bis zu etwa 300 GHz. Ferner bezieht sich die Bezeichnung "Hochgeschwindigkeits-Datensignale" auf Digitaldatenübertragungsraten von etwa 100 Kbit/s oder mehr, vorzugsweise Übertragungsraten von etwa 100 Mbit/s oder mehr, besonders bevorzugt Übertragungsraten von etwa 100 Gbit/s und sogar noch darüber. Auf diese Weise ist das Koaxialkabel geeignet, beispielsweise Bilder mit sehr hoher Qualität via UHF, digitales Video und/oder digitale HDTV-Signale zu übertragen.

Die Übertragung von Hochfrequenz- und/oder Hochgeschwindigkeits-Datensignalen ist besonders störanfällig. Insbesondere im medizinisch-technischen Bereich ist es jedoch häufig zwingend notwendig, dass Daten störungsfrei übertragen werden. Andernfalls besteht die Gefahr ernsthafter Komplikationen, beispielsweise wenn Mess- und/oder Steuerdaten von Lebenserhaltungsapparaturen fehlerhaft übertragen werden. Aus diesem Grund reicht eine einfache Schirmung der Seele des Koaxial-kabels in der Regel für den Einsatz im medizinisch-technischen Bereich nicht aus. Es werden stattdessen mehrere Schirme benötigt, um den Datenstrom in der Seele des Koaxialkabels vor äußeren elektromagnetischen Einflüssen, die zu einer Störung der Datensignalübertragung führen können, ausreichend zu schützen. Es sind auf dem Markt von einigen Anbietern bereits Koaxialkabel erhältlich, die eine Mehrzahl an Schirmen aufweisen. Diesen bekannten Koaxialkabeln ist gemein, dass sie mit einer massiven Seele versehen sind.

In OP-Sälen oder in Krankenhauszimmern ist es häufig von Vorteil, eine medizinisch-technische Apparatur beweglich an einer Haltevorrichtung, beispielsweise an einem Deckenstativ, zu lagern, um ein an der Haltevorrichtung befestigtes medizinisch-technisches Gerät - je nach Bedarf - in eine geeignete Position relativ zu einem Patienten und/oder zu einer anderen Person, z.B. dem behandelnden Arzt, verbringen zu können. Ein mit dem Gerät verbundener Rechner kann dabei abseits der Haltevorrichtung angeordnet und über ein entsprechendes Koaxialkabel mit dem Gerät verbunden sein, insbesondere um Hochfrequenz- und/oder Hochgeschwindigkeits-Datensignale mit dem Gerät auszutauschen. Eine Verlagerung des Geräts im Raum führt jedoch häufig dazu, dass sich das Koaxialkabel - wenngleich wohlmöglich nur gering - verformt. Dasselbe trifft zu, wenn die Apparatur am Ende eines Federarms angebracht ist, welcher eine gewisse Eigenelastizität aufweist. Verläuft das Koaxialkabel in oder an dem Federarm, ist das Koaxialkabel Verformungen ausgesetzt, die durch Erschütterungen des Federarms bedingt sind.

Es wurde nunmehr beobachtet, dass, insbesondere bei wiederholter Verlagerung eines an einer zuvor beschriebenen Haltevorrichtung befestigten medizinischtechnischen Geräts, ein an einem Ausleger oder Federarm der Haltevorrichtung angebrachtes, auf dem Markt erhältliches Koaxialkabel mit mehrfacher Schirmung und massiver Seele über die Zeit hinweg an der Seele derart ermüdet bzw. verschleißt, dass Störungen bei der Übertragung von Hochfrequenz- und/oder Hochgeschwindigkeits-Datensignalen auftreten.

Aus der EP 0 170 159 A2 ist ein konzentrisches Dreileiterkabel bekannt, das einen Innenleiter und zwei Außenleiter aus geflochtener Litze umfasst, die voneinander und von dem Innenleiter durch Isoliermaterial distanziert sind. Die Außenleiter bestehen jeweils aus mehreren Schichten, deren Gleichstromwiderstand mehrfach kleiner als der des Innenleiters ist.

Es ist daher Aufgabe der vorliegenden Erfindung, das zuvor genannte Problem zu lösen, und die störungsfreie Übertragung von Hochfrequenz- und/oder Hochgeschwindigkeits-Datensignalen im medizinisch-technischen Bereich zuverlässig, und möglichst dauerhaft, sicherzustellen. Insbesondere soll eine entsprechende Datenübertragung von oder zu einem an einer Haltevorrichtung beweglich gelagerten Gerät zuverlässig sichergestellt werden.

Diese Aufgabe wird gelöst durch die Merkmale des unabhängigen Patentanspruchs 1. Vorteilhafte Weiterbildungen sind Gegenstand der abhängigen Patentansprüche.

Nach einem ersten Aspekt der vorliegenden Erfindung wird die Aufgabe gelöst durch ein Koaxialkabel zur elektrischen Übertragung eines Hochfrequenz- und/oder Hoch geschwindigkeits-Datensignals, insbesondere für medizinisch-technische Anwendungen, umfassend eine radial innen angeordnete Seele und eine Mehrzahl von Schirmen, die die Seele radial außen umgeben, wobei die Seele eine Litze mit einer Mehrzahl von Einzeldrähten aufweist. Wie dies von den auf dem Markt erhältlichen Koaxialkabeln mit mehrfacher Schirmung bekannt ist, weisen die Schirme vorzugsweise unterschiedliche Innendurchmesser auf, wobei sie vorzugsweise die Seele konzentrisch umgeben. Die Schirme sind dabei in radialer Richtung direkt an aneinanderlegend angeordnet.

Indem die Seele eine Litze mit einer Mehrzahl von Einzeldrähten aufweist, lässt sich diese merklich einfacher biegen als eine massive Seele. Das erfindungsgemäße Koaxialkabel wird somit insgesamt flexibler und unterliegt auch bei wiederholten Verformungen keinen nennenswerten Ermüdungs- bzw. Verschleißerscheinungen. Durch die erfindungsgemäße Kombination der mehrfachen Schirmung und der im Wesentlichen ermüdungs- bzw. verschließfreien Seele kann dauerhaft eine störungsfreie Übertragung von Hochfrequenz- und/oder Hochgeschwindigkeits-Datensignalen sichergestellt werden.

Ferner ist vorzugsweise zwischen der Seele und den Schirmen wenigstens ein Dielektrikum vorgesehen und/oder sind die Schirme radial außen von einer Isolationsschicht bzw. Isolierung ummantelt.

Wenn die Haltevorrichtung für medizinisch-technische Geräte eine Gelenkstelle aufweist, die ein Schwenken bzw. Drehen von Abschnitten der Haltevorrichtung in einem nicht unerheblichen Winkelbereich, beispielsweise um mehr als 20°, erlaubt, so ist es vorteilhaft, wenn das Koaxialkabel an wenigstens einem seiner beiden Längsenden ein erstes Steckerteil aufweist, welches eingerichtet ist, mit einem komplementär ausgebildeten zweiten Steckerteil eine ununterbrochene elektrische Verbindung sicherzustellen, insbesondere während einer Relativdrehung der beiden Steckerteile um ihre jeweiligen Längserstreckungsachsen. Es ist somit möglich, in der Gelenkstelle der Haltevorrichtung durch zwei entsprechende Koaxialkabel eine elektrische Drehkupplung zu integrieren, so dass die einzelnen Koaxialkabel durch ein Schwenken oder Drehen der Haltevorrichtung um ihre Gelenkstelle kaum oder gar nicht verformt werden. Auf diese Weise kann der Verschleiß der Koaxialkabel weiter reduziert und eine störungsfreie Signalübertragung sichergestellt werden.

Unter dem Begriff "Steckerteil" ist gemäß der vorliegenden Erfindung sowohl ein männliches Steckerteil, d.h. der eigentliche Stecker, als alternativ auch das weibliche Gegenstück, d.h. die zum Stecker passende Buchse, zu verstehen.

Besonders vorteilhaft ist das Vorsehen eines zuvor beschriebenen Steckerteils, wenn die Relativdrehung wenigstens 90°, vorzugsweise wenigstens 180°, besonders bevorzugt wenigstens 360°, beträgt. Je größer der Winkelbereich ist, desto freier lässt sich die medizinisch-technische Vorrichtung im Raum platzieren.

Bei einer Relativverdrehung der beiden Steckerteile kommt es jedoch zwischen diesen zu Reibung, was in Folge wieder zu einem Materialverschleiß und somit zu einer möglichen Störung bei der Datenübertragung führt. Es wird daher bevorzugt, dass das Steckerteil eine elektrisch leitende, die Verschleißfestigkeit des Steckerteils erhöhende Beschichtung aufweist. Insbesondere sollten dabei zumindest die Oberflächenbereiche des Steckerteils entsprechend beschichtet sein, die mit Oberflächenbereichen des komplementär ausgebildeten Steckerteils bei der bestimmungsgemäßen Verbindung der beiden Steckerteile in direkten Kontakt gelangen.

Nach einem weiteren Aspekt betrifft die Erfindung eine elektrische Drehkupplung, umfassend zwei erfindungsgemäße Koaxialkabel, wobei ein erstes der beiden Koaxialkabel ein erstes Steckerteil aufweist, und wobei ein zweites der beiden Koaxialkabel ein zweites Steckerteil aufweist, welches komplementär zu dem ersten Steckerteil ausgebildet ist, wobei das erste und das zweite Steckerteil elektrisch leitend und relativ zueinander verdrehbar miteinander verbunden sind. Durch eine solche elektrische Drehkupplung ist es möglich, Verformungen von den einzelnen Koaxialkabeln weitgehend fernzuhalten, und somit den Verschleiß zu reduzieren, was wiederum eine dauerhaft störungsfreie Signalübertragung erlaubt.

Nach einem noch weiteren Aspekt betrifft die Erfindung eine Haltevorrichtung für medizinisch-technische Geräte, umfassend wenigstens einen um eine Gelenkstelle dreh- bzw. schwenkbar gelagerten Federarm oder Ausleger, wobei der Federarm oder Ausleger in der Gelenkstelle eine zuvor beschriebene Drehkupplung aufweist.

Vorzugsweise ist die Haltevorrichtung als Deckenstativ ausgebildet, so dass der Bonden des OP-Saals oder des Krankenhauszimmers frei gehalten und somit beispielsweise besser gereinigt werden kann.

Die Erfindung wird nachfolgend beispielhaft anhand der Figuren näher erläutert. Es stellt dar:
- Fig. 1: eine Seiten- und eine Querschnittsansicht eines erfindungsgemäßen Koaxialkabels,
- Fig. 2: ein Steckerteil für das in Fig. 1 dargestellte Koaxialkabel, und
- Fig. 3: ein Deckenstativ für medizinisch-technische Geräte, umfassend mehrere erfindungsgemäße Koaxialkabel.

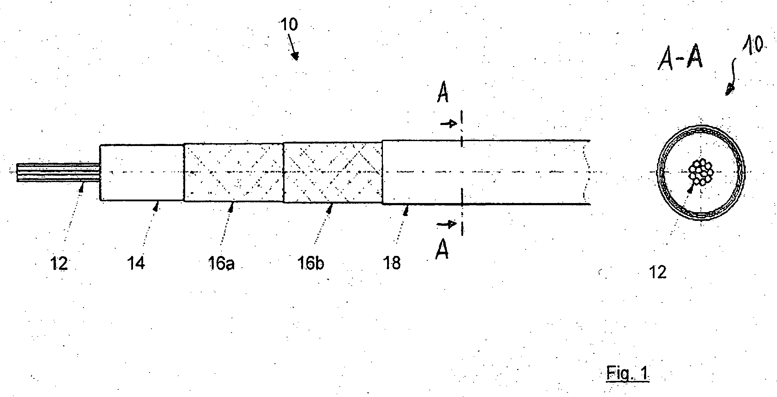

Links in Figur 1 ist eine Seitenansicht eines erfindungsgemäßen Koaxialkabels 10 dargestellt, wobei zur besseren Veranschaulichung seines Aufbaus einzelne Schichten des Koaxialkabels 10 abschnittsweise freigelegt sind. Rechts in Figur 1 ist eine dem angedeuteten Schnitt A-A entsprechende Querschnittsansicht des Koaxialkabels 10 zu erkennen. Das Koaxialkabel 10 weist um seine Mittelachse einen im Wesentlichen konzentrischen Aufbau auf. Im Zentrum dieses Aufbaus befindet sich eine Seele 12, die eine Litze mit einer Mehrzahl von Einzeldrähten aufweist. Hierdurch ist das erfindungsgemäße Koaxialkabel besonders biegsam bzw. flexibel und unterliegt bei Verformungen kaum Verschleiß- bzw. Ermüdungserscheinungen. Die Seele 12 ist radial außen von einem Dielektrikum 14 umgeben. Dieses ist wiederum radial außen von zwei Schirmen 16a und 16b umgeben. Die Schirme können wahlweise zum Beispiel aus einer Folie oder aus einem Drahtnetz gebildet sein. Die doppelte Schirmung ermöglicht eine zuverlässige elektrische Übertragung eines Hochfrequenz- und/oder HochgeschwindigkeitsDatensignals, und damit den Einsatz solcher Koaxialkabel für die Übertragung sensibler Signale im medizinischen Bereich.

Als radial äußerste Schicht weist das Koaxialkabel 10 eine Isolierung 18 auf.

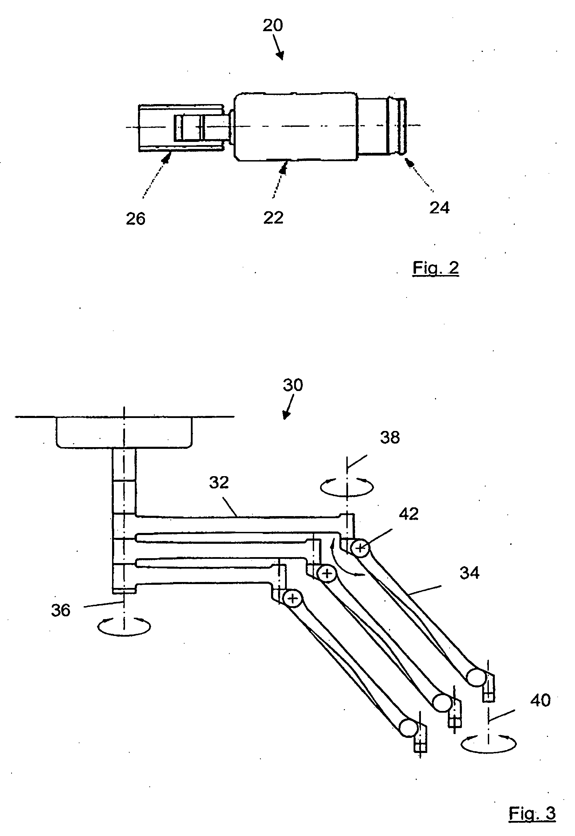

Figur 2 zeigt ein Steckerteil 20, welches dazu ausgebildet ist, mit einem Längsende des in Figur 1 dargestellten Koaxialkabels 10 dauerhaft durch Bördeln verbunden zu werden. Bei dem in Figur 2 dargestellten Steckerteil 20 handelt es sich um ein männliches Steckerteil (die komplementär ausgebildete Buchse ist hier nicht dargestellt). Das Steckerteil 20 umfasst einen Hauptkörper 22 mit einem Verbindungsabschnitt 24, dessen Außenoberfläche bei einer bestimmungsgemäßen Verbindung des Steckerteils 20 mit einer komplementär ausgebildeten Buchse mit Oberflächenabschnitten der Buchse in direkten Kontakt gelangt, um die elektrischen Signale zu übertragen. Das Steckerteil 20 ist im Wesentlichen rotationssymmetrisch in Bezug auf seine Längserstreckungsachse ausgebildet. Eine Drehung des Steckerteils 20 relativ zu der komplementär ausgebildeten Buchse ist somit bei einer bestimmungsgemäßen Verbindung von Steckerteil 20 und Buchse möglich, ohne dass dabei die Signalübertragung unterbrochen wird.

Die jeweiligen Kontaktflächen, mit denen das Steckerteil 20 bei bestimmungsgemäßer Verwendung in direkten Kontakt mit dem komplementär ausgebildeten Gegensteckerteil gelangt, sind dabei beschichtet, um die elektrische Leitfähigkeit und/oder die Verschleißeigenschaften der Steckerteile zu verbessern.

Am dem Verbindungsabschnitt 24 entgegengesetzten Längsende des Steckerteils 20 weist dieses einen Fortsatz auf, der dazu bestimmt ist, einen abisolierten kurzen Endabschnitt der Seele des Koaxialkabels (nicht dargestellt) in sich aufzunehmen. Um das Koaxialkabel dauerhaft mit dem Steckerteil derart zu verbinden, dass beide eine Einheit bilden, ist eine Crimphülse 26 vorgesehen. Diese kann, wenn der abisolierte Endabschnitt der Seele des Koaxialkabels in den Fortsatz des Steckerteils 20 eingeführt ist, durch eine Zange derart plastisch verformt werden, dass eine nicht mehr lösbare Verbindung zwischen dem Koaxialkabel und dem Steckerteil 20 entsteht.

Figur 3 zeigt ein Deckenstativ 30 für medizinisch-technische Geräte, welches drei im Wesentlichen identisch ausgebildete Ausleger aufweist. Jeder Ausleger umfasst einen ersten Auslegerabschnitt 32 und einen zweiten Auslegerabschnitt 34, wobei der zweite Auslegerabschnitt 34 alls - bedingt elastischer - Federarm ausgebildet ist. An dem freien Längsende eines jeden Auslegers befindet sich eine Kopplungsstelle zum Anbringen eines (hier nicht dargestellten) medizinisch-technischen Geräts, beispielsweise eines Monitors. Jeder Ausleger weist mehrere Drehgelenkstellen auf, nämlich um die Drehachsen 36, 38, 40 und 42 angeordnete Drehgelenkstellen, so dass sich ein an dem Ausleger befestigtes medizinisch-technisches Gerät relativ frei im Raum verlagern lässt.

Innerhalb eines jeden Auslegers ist wenigstens ein erfindungsgemäßes Koaxialkabel (wie in Figur 1 gezeigt) angeordnet, um ein an dem Ausleger befestigtes medizinisch-technisches Gerät beispielsweise mit einem abseits des Deckenstativs 30 platzierten Computer zu verbinden. Bei einer Bewegung eines Auslegers im Raum oder durch auf den elastischen Federarm 34 wirkende Erschütterungen ist das Koaxialkabel wiederholt Verformungen ausgesetzt. An den Gelenkstellen, an denen die Verformungen des Koaxialkabels bei Drehung um die Gelenkstelle besonders ausgeprägt sind, beispielsweise an den Drehgelenkstellen um die vertikalen Achsen 36, 38 und 40, ist es vorteilhaft, eine elektrische Drehkupplung innerhalb der Gelenkstelle vorzusehen. Beispielsweise könnte ein erstes Koaxialkabel (wie in Figur 1 gezeigt) in dem ersten Auslegerabschnitt 32 entlang dessen Längserstreckungsrichtung verlegt sein, wohingegen ein zweites Koaxialkabel in dem zweiten Auslegerabschnitt 34 entlang dessen Längserstreckungsrichtung verlegt sein könnte. An der Drehgelenkstelle um die Achse 38 könnte ein erstes Steckerteil des ersten Koaxialkabels in Eingriff mit einem komplementär ausgebildeten zweiten Steckerteil des zweiten Koaxialkabels stehen, um auf diese Weise eine elektrische Drehkupplung zu bilden.

Durch den Einsatz von erfindungsgemäßen Koaxialkabeln in den Auslegerarmen des Deckenstativs 30 und - bei Bedarf - durch die Bildung von elektrischen Drehkupplungen an den Gelenkstellen der Auslegerarme, wird eine dauerhaft störungsfreie Übertragung von Hochfrequenz- und/oder Hochgeschwindigkeits-Datensignalen von oder zu medizinisch-technischen Geräten gewährleistet.

## Patentansprüche

1. Koaxialkabel (10) zur elektrischen Übertragung eines Hochfrequenz- und/oder Hochgeschwindigkeits-Datensignals, insbesondere für medizinisch-technische Anwendungen, umfassend eine radial innen angeordnete Seele (12) und eine Mehrzahl von Schirmen (16a, 16b), die die Seele (12) radial außen umgeben, wobei
die Seele (12) eine Litze mit einer Mehrzahl von Einzeldrähten aufweist, wobei die Schirme (16a, 16b) unterschiedliche Innendurchmesser aufweisen und die Seele (12) konzentrisch umgeben, **dadurch gekennzeichnet, dass** genau zwei Schirme (16a, 16b) vorgesehen sind, die in radialer Richtung direkt aneinander liegen.

2. Koaxialkabel (10) nach Anspruch 1, wobei zwischen der Seele (12) und den Schirmen (16a, 16b) wenigstens ein Dielektrikum (14) vorgesehen ist.

3. Koaxialkabel (10) nach einem der vorhergehenden Ansprüche, wobei die Schirme (16a, 16b) radial außen von einer Isolationsschicht (18) ummantelt sind.

4. Koaxialkabel (10) nach einem der vorhergehenden Ansprüche, wobei das Koaxialkabel (10) an wenigstens einem seiner beiden Längsenden ein erstes Steckerteil (20) aufweist, welches eingerichtet ist, mit einem komplementär ausgebildeten zweiten Steckerteil eine ununterbrochene elektrische Verbindung sicherzustellen, insbesondere während einer Relativdrehung der beiden Steckerteile um ihre jeweiligen Längserstreckungsachsen.

5. Koaxialkabel (10) nach Anspruch 5, wobei die Relativdrehung wenigstens 90°, vorzugsweise wenigstens 180°, besonders bevorzugt wenigstens 360°, beträgt.

6. Koaxialkabel (10) nach Anspruch 4 oder 5, wobei das Steckerteil (20) eine elektrisch leitende, die Verschleißfestigkeit des Steckerteils erhöhende Beschichtung aufweist.

7. Koaxialkabel (10) nach Anspruch 4, 5 oder 6, wobei das Steckerteil (20) im Wesentlichen rotationssymmetrisch ist und durch einen Hauptkörper (22) mit einem Verbindungsabschnitt (24) und einen am dem Verbindungsabschnitt (24) entgegengesetzten Längsende des Steckerteils (20) vorgesehenen Fortsatz mit einer Crimphülse (26) gebildet ist.

8. Elektrische Drehkupplung, umfassend zwei Koaxialkabel (10) nach einem der vorhergehenden Ansprüche, wobei ein erstes der beiden Koaxialkabel (10) ein erstes Steckerteil (20) aufweist, und wobei ein zweites der beiden Koaxialkabel ein zweites Steckerteil aufweist, welches komplementär zu dem ersten Steckerteil (20) ausgebildet ist, wobei das erste und das zweite Steckerteil elektrisch leitend und relativ zueinander verdrehbar miteinander verbunden sind.

9. Haltevorrichtung, insbesondere Deckenstativ (30), für medizinisch-technische Geräte, umfassend wenigstens einen um eine Gelenkstelle drehbar oder schwenkbar gelagerten Federarm oder Ausleger (32, 34), wobei der Federarm oder Ausleger (32, 34) in der Gelenkstelle eine elektrische Drehkupplung nach Anspruch 8 aufweist.

## Claims

1. Coaxial cable (10) for electrical transmission of a high-frequency and/or highspeed data signal, in particular for medical-engineering applications, comprising a core (12) arranged radially inside and a plurality of shields (16a, 16b) which surround the core (12) radially outside, wherein
the core (12) exhibits a litz with a plurality of individual wires, wherein the shields (16a, 16b) have differing inner diameters and surround the core (12) concentrically, **characterized in that** exactly two screens (16a, 16b) are provided that are directly adjoining each other in radial direction.

2. Coaxial cable (10) according to Claim 1, wherein at least one dielectric (14) is provided between the core (12) and the shields (16a, 16b).

3. Coaxial cable (10) according to one of the preceding claims, wherein the shields (16a, 16b) are jacketed radially outside by an insulating layer (18).

4. Coaxial cable (10) according to one of the preceding claims, wherein the coaxial cable (10) exhibits, at at least one of its two longitudinal ends, a first plug member (20) which is adapted to ensure an uninterrupted electrical connection to a second plug member of complementary design, in particular during a relative rotation of the two plug members about their respective axes of longitudinal extent.

5. Coaxial cable (10) according to Claim 4, wherein the relative rotation amounts to at least 90°, preferentially at least 180°, particularly preferably at least 360°.

6. Coaxial cable (10) according to Claim 4 or 5, wherein the plug member (20) exhibits an electrically conducting, the wear resistance increasing coating of the plug member.

7. Coaxial cable (10) according to Claim 4, 5 or 6, wherein the plug member (20) is substantially rotationally symmetrical and is formed by a main body (22) with a connecting portion (24) and by an extension, provided on the longitudinal end of the plug member (20) opposite the connecting portion (24), with a crimp sleeve (26).

8. Electrical rotary coupling comprising two coaxial cables (10) according to one of the preceding claims, a first of the two coaxial cables (10) exhibiting a first plug member (20), and a second of the two coaxial cables exhibiting a second plug member, which is designed to be complementary to the first plug member (20), the first and the second plug members being connected to one another electrically conducting and capable of twisting relative to one another.

9. Holding device, in particular ceiling support (30), for medical-engineering instruments, comprising at least one spring arm or jib (32, 34) that is supported so as to be capable of rotating or swivelling about a hinge point, the spring arm or jib (32, 34) in the hinge point exhibiting an electrical rotary coupling according to Claim 8.

## Revendications

1. Câble coaxial (10) pour la transmission électrique d'un signal de données haute fréquence et/ou haute vitesse, en particulier pour des applications techniques en médecine, comprenant une âme (12) disposée radialement côté intérieur et une pluralité de blindages (16a, 16b), qui entourent radialement côté extérieur l'âme (12), l'âme (12) présentant un toron muni d'une pluralité de fils individuels, tandis que les blindages (16a, 16b) présentent des diamètres intérieurs différents et entourent de manière concentrique l'âme (12), **caractérisé en ce que** sont prévus précisément deux blindages (16a, 16b), qui reposent directement l'un contre l'autre dans une direction radiale.

2. Câble coaxial (10) selon la revendication 1, dans lequel au moins un diélectrique (14) est prévu entre l'âme (12) et les blindages (16a, 16b).

3. Câble coaxial (10) selon l'une quelconque des revendications précédentes, dans lequel les blindages (16a, 16b) sont enveloppés radialement côté extérieur d'une couche isolante (18).

4. Câble coaxial (10) selon l'une quelconque des revendications précédentes, dans lequel le câble coaxial (10) présente, au niveau au moins de l'une de ses deux extrémités longitudinales, une première partie enfichable (20), qui est conçue pour assurer un raccordement électrique ininterrompu avec une deuxième partie enfichable réalisée de manière complémentaire, en particulier au cours d'une rotation relative des deux parties enfichables autour de leurs axes d'extension longitudinale respectifs.

5. Câble coaxial (10) selon la revendication 4, dans lequel la rotation relative est égale à au moins 90°, de préférence à au moins 180°, de manière particulièrement préférée à au moins 360°.

6. Câble coaxial (10) selon la revendication 4 ou 5, dans lequel la partie enfichable (20) présente un revêtement électroconducteur améliorant la résistance à l'usure de la partie enfichable.

7. Câble coaxial (10) selon la revendication 4, 5 ou 6, dans lequel la partie enfichable (20) est sensiblement symétrique en rotation et est formée par un corps principal (22) pourvu d'un tronçon de raccordement (24) et par un appendice, prévu au niveau de l'extrémité longitudinale, opposée au tronçon de raccordement (24), de la partie enfichable (20), pourvu d'un manchon à sertir (26).

8. Couplage rotatif électrique, comprenant deux câbles coaxiaux (10) selon l'une quelconque des revendications précédentes, dans lequel un premier des deux câbles coaxiaux (10) présente une première partie enfichable (20) et dans lequel un second des deux câbles coaxiaux présente une deuxième partie enfichable, laquelle est réalisée de manière complémentaire à la première partie enfichable (20), dans lequel la première et la deuxième partie enfichable sont raccordées entre elles de manière électroconductrice et de manière à pouvoir tourner l'une par rapport à l'autre.

9. Dispositif de maintien, en particulier support fixé au plafond (30), pour des appareils techniques utilisés en médecine, comprenant au moins un bras à ressort ou un avant-bras (32, 34) monté de manière à pouvoir tourner ou pivoter autour d'un point d'articulation, dans lequel le bras à ressort ou l'avant-bras (32, 34) présente, dans le point d'articulation, un couplage rotatif électrique selon la revendication 8.
